# EUROPEAN PATENT SPECIFICATION

(11) **EP 0 307 011 B1**
(45) Date of publication and mention of the grant of the patent: **07.09.1994**
(21) Application number: 88201203.2
(22) Date of filing: 13.06.1988
(51) Int. Cl.: C12N 9/52, C12N 9/64, C12N 15/52, C12N 15/74

(54) **DNA fragments, containing a lactic acid bacterium-specific regulator region for the expression of genes coding for normally heterologous proteins**
DNA-Fragmente, die ein für Milchsäurebakterien spezifisches Regulationsgebiet enthalten, zur Expression von Genen, die normalerweise heterologe Proteine kodieren
Fragments d'ADN contenant une région régulatrice, spécifique pour les bactéries lactiques, pour l'expression de gènes encodant des protéines normalement hétérologues

(30) Priority: 12.06.1987 NL 8701378
(43) Date of publication of application: 15.03.1989
(73) Proprietor: NEDERLANDS INSTITUUT VOOR ZUIVELONDERZOEK, 6710 BA Ede (Gld) (NL)
(72) Inventor: Simons, Augustinus Franciscus Maria, NL-6715 JP Ede (NL); De Vos, Willem Meindert, NL-6714 MD Ede (NL)
(74) Representative: Baarslag, Aldert D.

(56) References cited:
- NETHERLANDS MILK AND DAIRY JOURNAL, vol. 40, no. 2/3, 1986, pages 141-154, Wageningen, NL; W.M. De VOS
- APPLIED AND ENVIRONMENTAL MICROBIOLOGY, vol. 50, no. 1, July 1985, pages 94-101; Am. Soc. for Microbiology; J. KOK et al.
- 101, American Society for Microbiology; J. KOK et al.: "Cloning and expressionof a Streptococcus cremoris proteinase in Bacillus subtilis and Streptococcuslactis"

## Description

The invention first of all relates to DNA fragments which contain a lactic acid bacterium-specific regulator region suitable for the substantial expression of genes coding for, in particular, heterologous proteins such as proteolytic enzymes, for example chymosin, as well as pseudo-, pro- and preprochymosin. It is known that chymosin is a proteolytic enzyme which is synthesized in the abomasum of young calves and is excreted as a zymogen, prochymosin, comprising 365 amino acids. Prochymosin is converted to chymosin by the removal of 42 amino-terminal amino acids by means of a pH-dependent autocatalytic process. More particularly, chymosin exists in the form of 2 isoenzymes (A and B). The two forms differ from each other in that an aspartic acid is present at position 290 in the A form and that a glycine residue is found at that position in the B form. This calf chymosin has already been used for a very long time in the preparation of cheese. However, the continued increase in cheese production and the decrease in the supply of calf stomachs have resulted in a shortage of calf chymosin. Therefore, use is made in many cases in cheese preparation of other enzymes of animal of microbial origin. However, in view of the superior quality of chymosin as compare with these other enzymes there is considerable interest in micro-organisms which can produce large quantities of prochymosin using recombinant DNA techniques.

Also with reference to the abovementioned cheese production it is mentioned that lactic acid bacteria such as Streptococcus lactis, Streptococcus cremoris, Streptococcus diacetylactis, Streptococcus thermophilus, Leuconostoc lactis, Leuconostoc cremoris and Lactobacillus spp are used on a large scale in the dairy industry for the production of fermented milk products such as cheese, yoghurt, butter and buttermilk. The growth of lactic acid bacteria depends on amino acids and peptides which are produced after decomposition of casein (the most important protein in cows' milk) under the influence of protein-splitting enzymes such as proteinases and peptidases. Proteinases decompose casein into small fractions followed by decomposition of the protein fractions by the peptidases into amino acids. The proteinases which are derived from various Streptococcus cremoris strains show a manner of decomposition of the various casein components αsl, β and k-casein, which is characteristic for such a strain. The industrial strain S.cremoris SK11 contains a cell wall-bound proteinase which decomposes αsl, β and k-casein. The unique caseinolytic specificity of this proteinase has the result that this strain does not lead to formation of bitter-tasting peptides from casein.

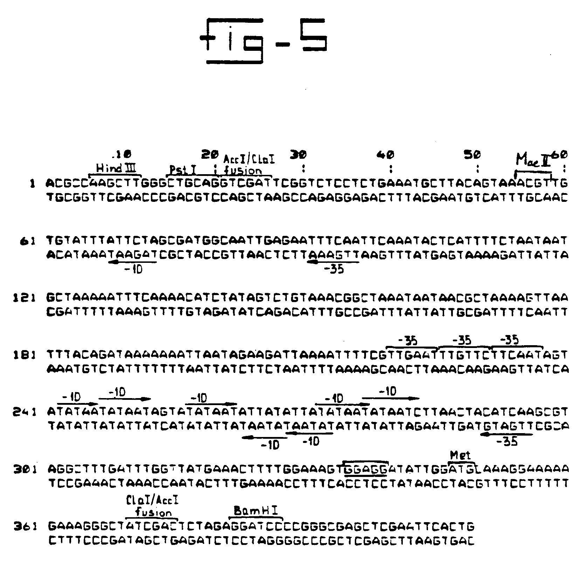

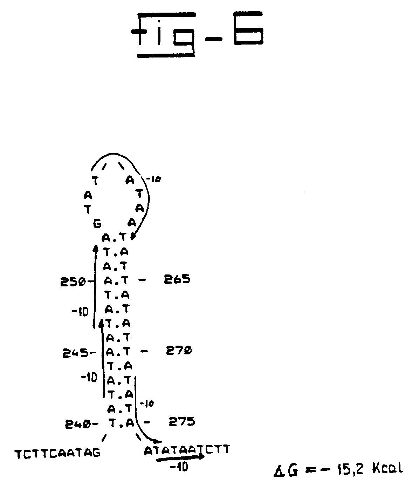

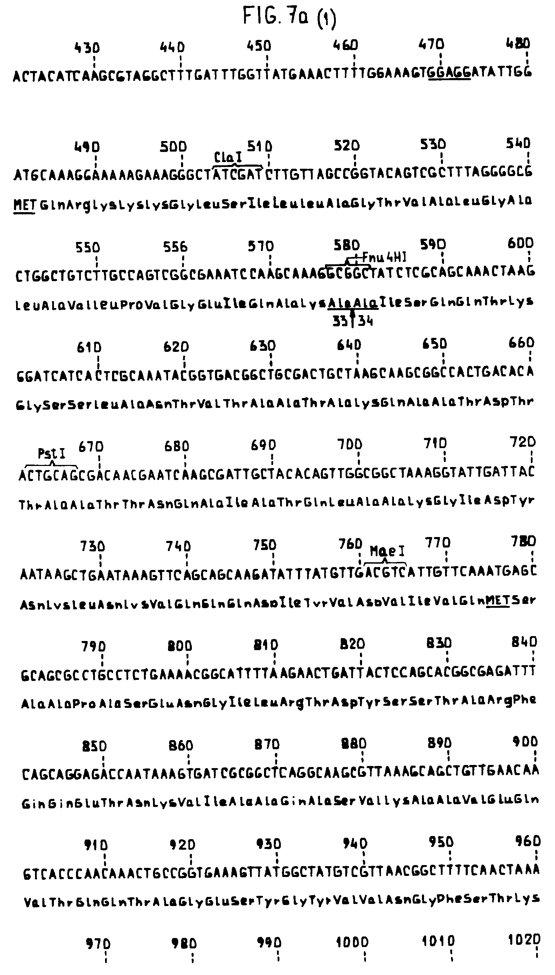

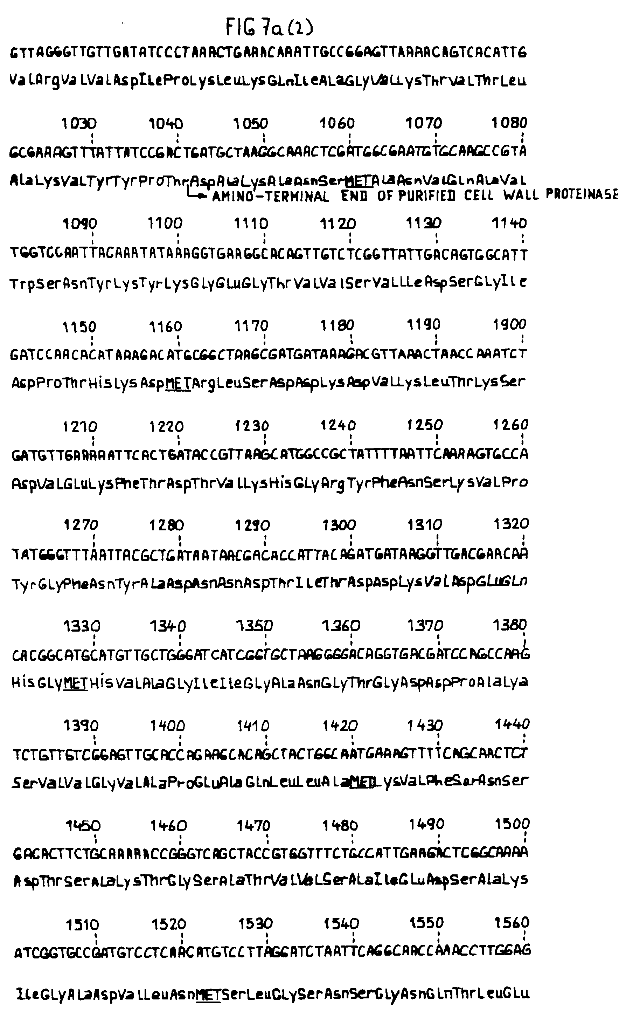

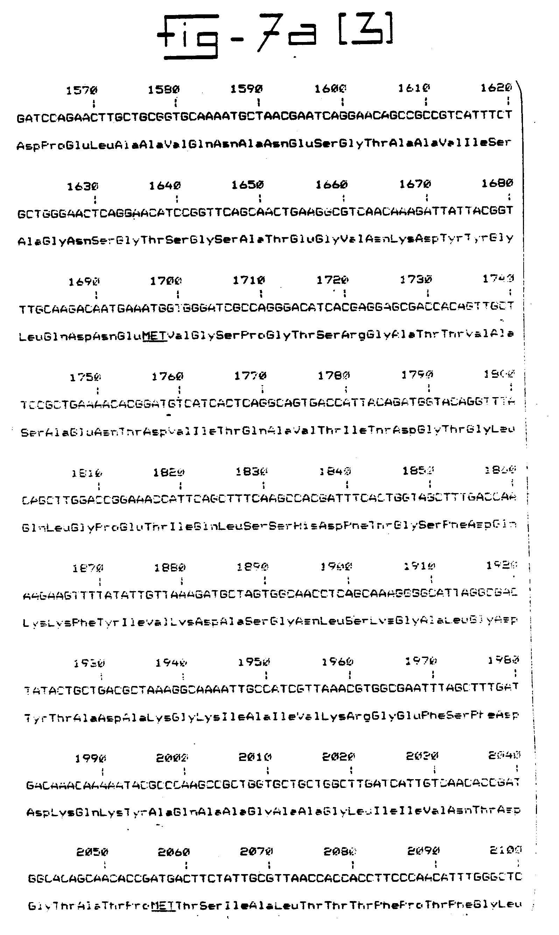

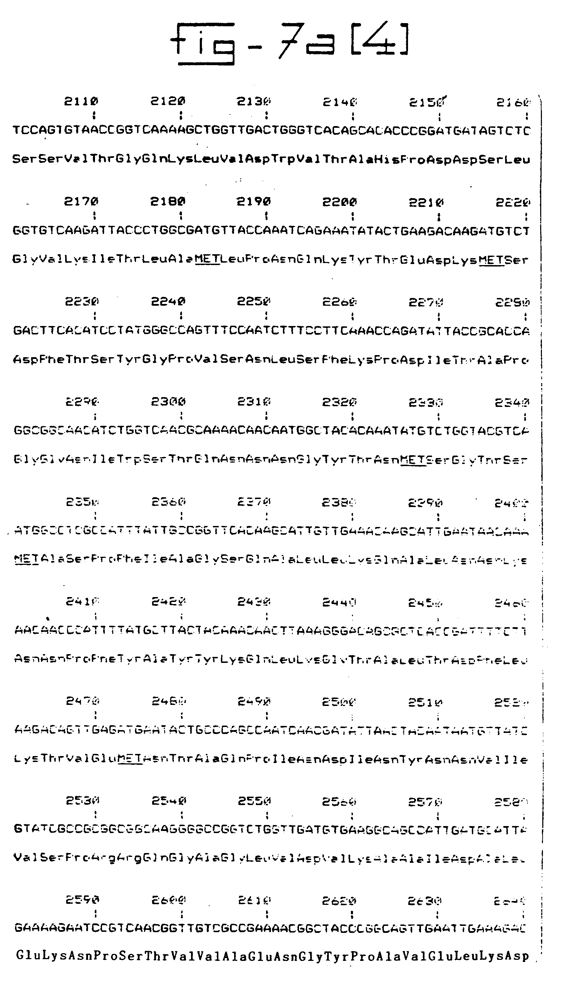

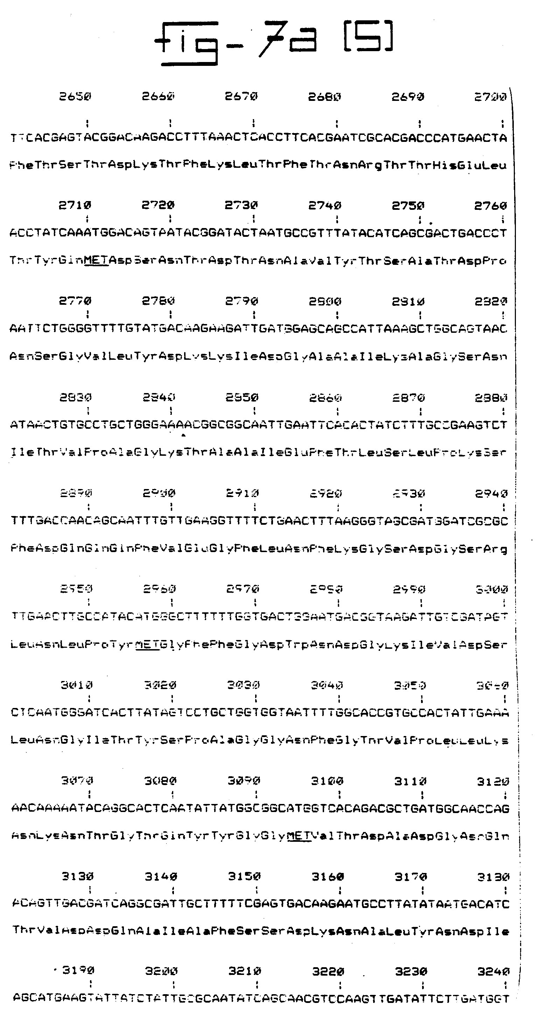

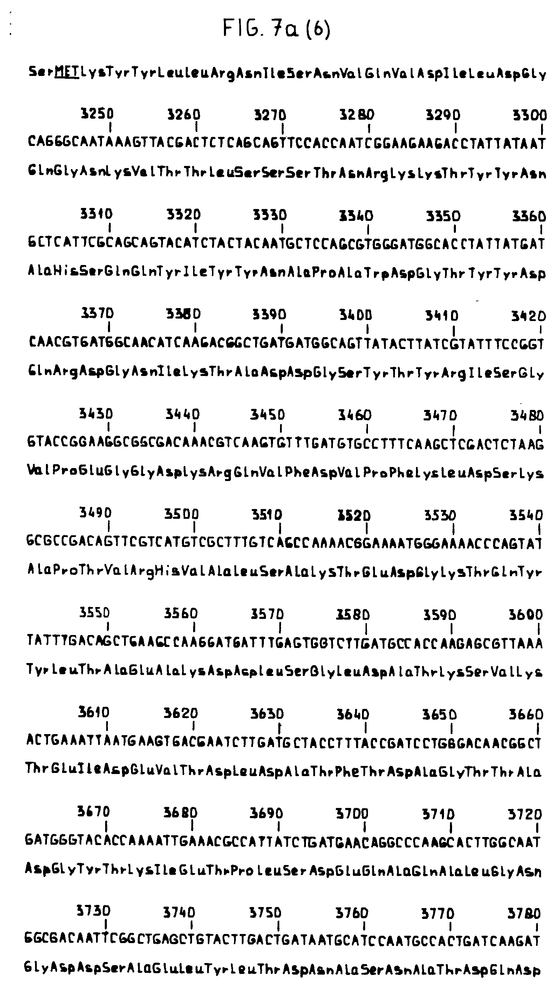

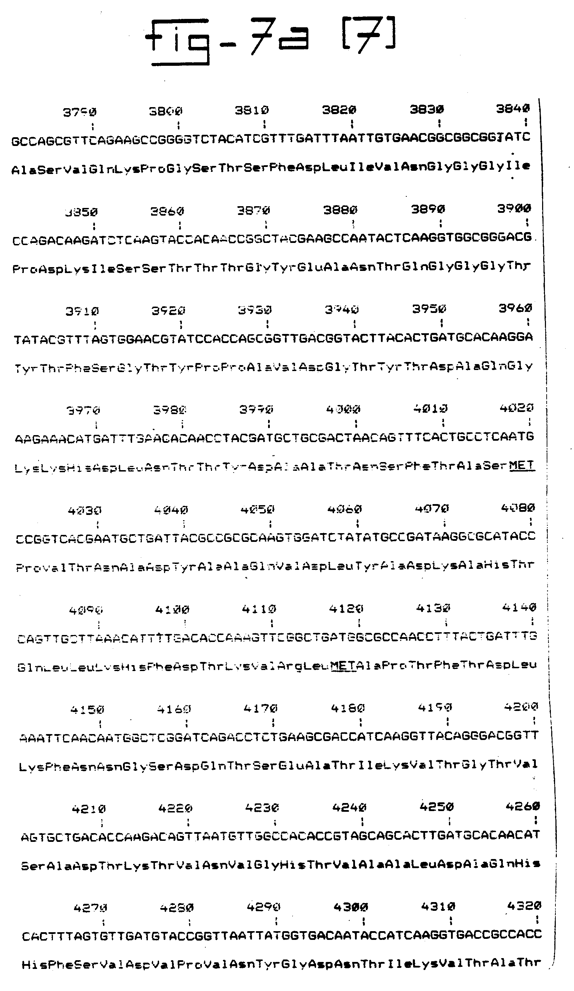

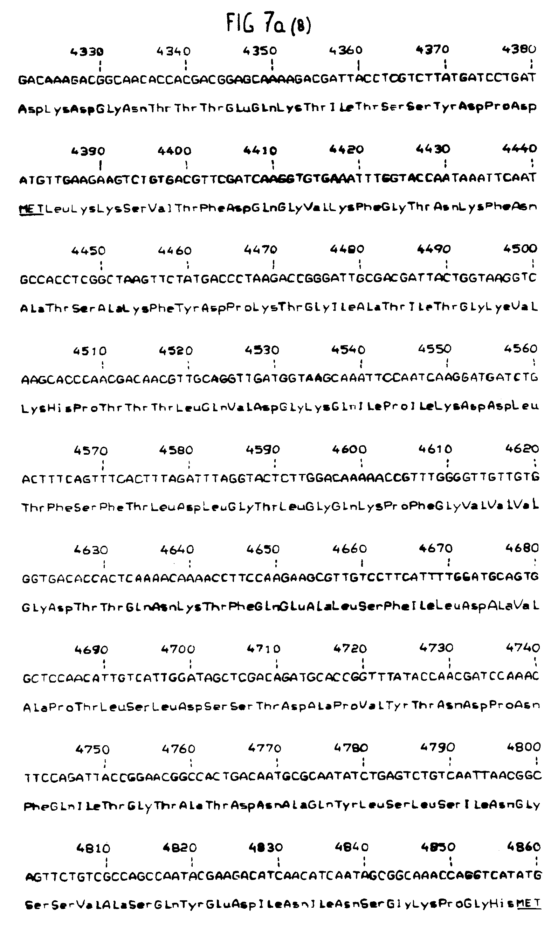

In relation to what has been said above, the invention relates to isolated DNA fragments containing the regulator region of the proteinase gene as shown in Figure 5 containing the ClaI fragment located on the plasmid pSK111 of the Streptococcus cremoris SK11 strain SK112, deposited at the Central Bureau for Mould Cultures, The Netherlands, under No. CBS 349.87 and mutated forms thereof having essentially the same activity. More in particular the invention relates to the ClaI fragment of the proteinase gene of the plasmid pSK111 of the strain S.cremoris SK112, as shown in Fig. 5, optionally supplemented by the DNA part-region shown in Fig. 7a and downstream of the ClaI fragment or by the DNA part-region shown in Fig. 7b and located upstream of the ClaI fragment. More in particular it has appeared that the fragment located in Fig. 7a(1) between the ClaI-site, present in the second line and the Ala-Ala-sequence, present in the third line of said figure, may procure the secretion of for instance prochymosin, if connected downstream of the ClaI fragment, illustrated in Fig. 5. Because of this, the host is capable to secrete the produced prochymosin into the medium which means a considerable advantage with respect to the situation of a protein brought into expression but remaining in the cell.

The invention secondly relates to the recombinant plasmids which are provided with a lactic acid bacterium-specific replicon and which, moreover, possess a wide host range and which replicate with a high or low copy number in lactic acid bacteria. The vectors pNZ121 and pNZ122 which are defined in detail below are mentioned as specific plasmid vectors.

The gene coding for prochymosin, preprochymosin, pseudochymosin or chymosin is advantageously used as the gene of a heterologous protein which is connected to a DNA regulator fragment according to the invention. The gene for β-galactosidase may be mentioned as a further example of a gene coding for a heterologous protein.

The invention furthermore relates to a recombinant plasmid which contains a combination of a plasmid vector, a DNA regulator region and a gene for a heterologous protein.

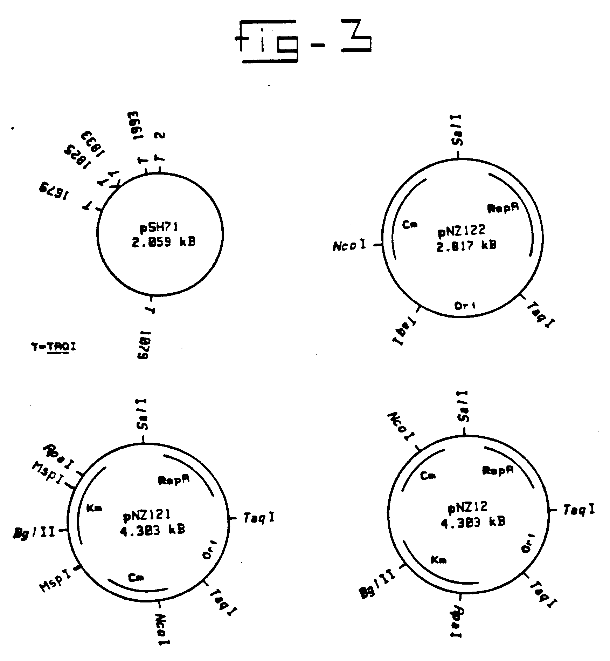

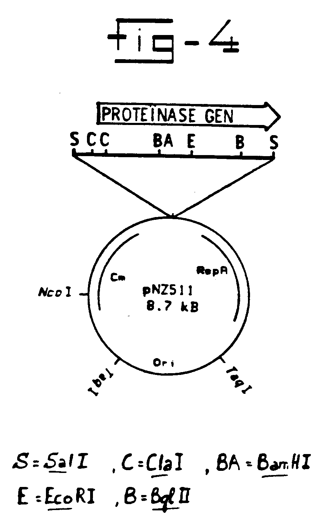

At the same time, a recombinant plasmid which contains a homologous gene and, in particular, a protinase gene, such as shown in Figure 4, can be used according to the invention. In this respect it is brought to the fore that contrary to the proteinase described in WO85/03945 the invention relates to a "non-bitter" proteinase (W.M. de Vos, Neth. Milk Dairy, 40, (1986), 14-154).

The invention furthermore relates to micro-organisms, in particular lactic acid bacteria, very particularly the lactic acid bacterium Streptococcus lactis MG1363, containing at least one recombinant plasmid according to the invention.

The invention finally also relates to the proteins and foodstuffs, such as cheese, etc. prepared with the abovementioned microorganisms.

The invention is illustrated with reference to
a) the prochymosin gene derived from a calf, and
b) the DNA fragment containing the promoter, the ribosome binding site and the sequence coding for a number of amino-terminal amino acid including the AUG initiator codon derived from the proteinase gene of the plasmid pSK111 of the strain S. cremoris SK11
and a plasmid, with the two elements mentioned under (a) and (b) as well as with reference to a lactic acid bacterium strain containing such a plasmid; this illustration is not to be regarded as being limiting, in particular not in respect of the dimensions of the DNA derived from the proteinase gene and the gene coding for the heterologous protein.

### Classification of the procedures followed:

The procedures below are followed for constructing microorganisms which are capable of producing calf chymosin.
A) Isolation of messenger-RNA.
   A messenger-RNA (mRNA) fraction was isolated from the mucosal cells of an abomasum of a young calf. The mRNA was separated from the other RNA constituents by means of oligo-dT cellulose column chromatography.
B) Synthesis of copy-DNA.
   For the preparation of copy-DNA (cDNA) against the isolated mRNA fraction in which the mRNA functions as matrix, purified mRNA was incubated in a suitable buffer with the enzyme reverse transcriptase, a primer and a mixture of the four deoxynucleotides (dNTP's). The RNA was subsequently removed by an alkaline treatment and the complementary strand was synthesized with the aid of the enzyme DNA-polymerase I and the four dNTP's. The hairpin in the double-stranded DNA produced was removed with the aid of S1 nuclease. The mixture of double-stranded DNA pieces was fractionated and only the fragments having a suitable length were ligated into a cloning vector.
C) Tailing and transformation.
   A widely used method for cloning of cDNA fragments in a suitable vector is the provision of fragments with poly C tails and at the same time provision of a suitable cloning vector, such as pUC9, with poly G tails utilizing the enzyme terminal transferase. Bacteria posessing plasmid pUC9 led, in a suitable host, to the synthesis of the enzyme β-galactosidase. Addition of the dyestuff 5-bromo-4-chloro-3-indolyl-β-D-galacto-pyranoside (X-gal) to the agar plates resulted in blue colonies. Transformed cells which possessed recombinant plasmids were, however, unable to synthesize β-galactosidase and therefore did not produce a blue coloration.
D) Isolation of the chymosin clone.
   Selection of those clones containing said recombinant plasmids containing the plasmid pUC9 and information for the calf chymosin took place by means of radio-labelled probes. On the basis of already published chymosin DNA sequences, it was possible to synthesize synthetic DNA probes specific for chymosin DNA. The white colonies on the agar plate were transferred to a nitrocellulose filter and the replica produced was treated with the suitable radioactive probe.
E) Characterization of chymosin clones.
   The selected colonies contain plasmids with the expected chymosin sequence which can subsequently be characterized by suitable restriction endonucleases. The complete DNA sequence of the chymosin information containing plasmids was determined with the aid of enzymatic DNA sequencing techniques and was related to the amino acid sequence of prochymosin.
F) Expression signals.
   Suitable DNA fragments containing the complete or virtually complete prochymosin information were subsequently to be placed under the control of functional expression signals and transformed into suitable hosts. Expression signals can be randomly isolated with the aid of promoter probe vectors or use can be made of expression signals of a defined origin. In view of the fact that proteineases are located on the outside of the cell wall, so as to bring about any secretion of the prochymosin, the choice was made to use expression signals derived from the proteinase gene of the strain S. cremoris SK11.
G) Location of the cell wall-bound proteinase gene.
   The mesophilic lactic acid bacteria used in cheese production generally contain, in addition to chromosomal DNA, also a large number (2-10) of plasmids. Although the majority of these plasmids has been found to be cryptic a few plasmids code entirely or partially for certain functions essential for the cell. Curing and transfer experiments have shown that the complete genetic information for the proteinease gene in strain S. cremoris SK11 is located on plasmid pSK111 (size 78 kb).
H) Cloning of the S. cremoris SK11 proteinase gene.
   Restriction enzyme fragments of the proteinase plasmid pSK111 were cloned in E. coli with the aid of bacteriophage lambda vectors. Screening of recombinant phages which synthesize proteinase took place via an immunological test in which use was made of antibodies directed against S. cremoris SK11 cell wall proteinase. The insertion DNA present in the positively reacting phages was characterized with the aid of restriction enzymes and compared with the physical map of pSK111. Location of the structural proteinase gene and the regulator region took place by isolating suitable restriction enzyme fragments from the recombinant phages and cloning in a proteinase-deficient S. lactis strain with the aid of lactobacillus-specific plasmid vectors. Transformants capable of growing in milk contained the complete genetic information for the proteinase, including the regulator region.
I) Determination of the DNA nucleotide sequence of the regulator region and the structural area of the proteinase gene of strain S. cremoris SK11.
   The total nucleotide sequence of the regulator and structural regions of the proteinase gene were deter mined. The functionality of the regulator region was determined by fusing the hypothetical promoter sequence and ribosome binding site with the decapitated E. coli β-galactosidase gene (lacZ) of plasmid pMC1403. This plasmid lacked the promoter region, the ribosome binding site and the eight amino-terminal amino acids of the lacZ gene. The amino-terminal end of the purified SK11 cell wall proteinase was subsequently determined and this was compared with the amino acid sequence derived from the DNA sequence of the structural gene.
J) Coupling of the proteinase promoter to the genetic information of prochymosin.
   The regulator region of the proteinase gene of strain S. cremoris SK11 was subsequently coupled to the genetic information of prochymosin. In the constructed plasmid, a prochymosin fusion protein was produced after expression of the genetic information in a suitable host. By providing the plasmid with an origin of replication region derived from lactic acid Streptococci it was possible to control the synthesis of this prochymosin fusion protein in S. lactis.
K) Demonstration of chymosin synthesis by lactic acid bacteria.
   This took place by means of an immunological screening method (Western blot). To do this, the content of a S. lactis cell extract (intracellular prochymosin) or the supernatant of a S.lactis culture (extracellular prochymosin) was analysed on an SDS polyacrylamide gel and subsequently transferred to a nitrocellulose filter (blotting). The filter was incubated with antibodies against chymosin, and the antigen/antibody complex could be made visible via an enzymatic reaction. Both qualitative and quantitative detection and identification of the synthesized product are possible with the aid of this method.

### Detailed description of the invention.

### A) Isolation of messenger RNA.

The muscosal tissue of a number of abomasums of calves which were about 1-2 weeks old and had just been slaughtered was scraped off and immediately thereafter processed further or frozen at -80°C. The tissue was subsequently taken up in a solution of guanidinium thiocyanate for protecting the mRNA against RNases (Chirgwin J.M. et al., Biochemistry 18, (1979), pages 5294-5299) and pulverized in a Potter homogenizer. The homogenate was centrifuged in a Sorvall GSA rotor at 10,000 rpm for 20 minutes at 4°C. The supernatant was mixed with 0.025 volume of 1 M acetic acid solution and 0.75 volume of absolute ethanol and left to stand overnight at -20°C. The precipitated nucleic acid was sedimented by centrifugation in a Sorvall GSA rotor at 10,000 rpm for 30 minutes at 4°C. The pellet was taken up in a guanidine hydrochloride solution and the RNA was precipitated by addition of 0.025 volume of 1 M acetic acid and 0.5 volume of ethanol, and the mixture was left to stand at -20°C for at least 3 hours. After centrifugation in a Sorvall SS34 rotor at 15,000 rpm for 20 minutes at 4°C, the guanidine hypochloride step was repeated once again and the ultimately obtained RNA product was subjected to oligo-dT cellulose chromatography (Aviv, H. and Leder, P., Proc. Natl. Acad. Sci. USA 69, (1972), pages 1408-1412). The RNA preparation was dissolved in a suitable buffer (0.5% SDS, 0.5 M NaCl, 0.01 M EDTA, 0.01 M Tris-HCl, pH = 7.4) in a concentration of 2 mg/ml, bound to the column and eluted with eluent buffer (0.5% SDS, 0.01 M Tris-HCl, pH = 7.4) and the polyA-containing mRNA fractions were precipitated with alcohol. The oligo-dT-cellulose column chromatography purification was repeated once again. Before the mRNA preparation was used as template for the in vitro DNA synthesis, a cell-free extract of rabbit reticulocytes (Pelham, J. and Jackson, R., Eur. J. Biochem. 67, (1976), pages 247-256) was incubated with mRNA in order to ensure that (prepro)chymosin mRNA molecules were present in the isolated mRNA fractions. Under the influence of the mRNA fraction, a protein having a molecular weight of 40,000 D, which is the expected size of the prochymosin, was synthesized in a cell-free system. In order to confirm that this band represents prochymosin the translation mixture was incubated in vitro with an antiserum against chymosin. Only the 40,000 D band was precipitated by the antiserum.

### B) Synthesis of copy-DNA.

The total mRNA preparation was converted into copy-DNA with the aid of the enzyme reverse transcriptase from the avian myeloblastosis virus (AMV) by the method described by Ulrich, A. et al., Science 196, (1977), pages 1313-1319. Ten µg of oligo-dT primer was added to ten micrograms of total mRNA, briefly heated at 90°C and immediately put on ice. Deoxynucleoside triphosphates (all four) were added to a final concentration of 0.5 mM followed by 60 units of RNasin (Promega) and 50 µCi α-32P- dATP in a buffer with a final concentration of 40 mM KCl, 6 mM MgCl₂ and 50 mM Tris-HCl of pH = 8.3. The reaction was started by addition of 100 units of reverse transcriptase (AMV) and incubation was carried out for 1 hour at 40°C. The reaction was stopped by addition of EDTA to a final concentration of 20 mM and brought to 50 mM with NaOH to decompose the RNA in the RNA-DNA duplex. After 1-hour incubation at 65°C, the solution was neutralized by addition of Tris-HCl of pH = 8.0 to a final concentration of 250 mM and HCl to 0.25 N. The reaction mixture was subsequently extracted with phenol/chloroform and the free unincorporated triphosphates were removed by gel filtration over Sephadex G-50. The single-stranded copy-DNA was subsequently precipitated with alcohol. The second DNA strand was synthesized with the aid of the enzyme DNA polymerase I (15 units) in the presence of 25 µCi of α -P32-dATP and deoxynucleoside triphosphates to a final concentration of 250 µm in a reaction buffer with, as final composition, 100 mM K phosphate of pH = 6.9, 5 mM DTT and 10 mM MgCl₂. The mixture was incubated at 15°C for 3 to 4 hours. After a phenol/chloroform extraction and Sephadex G-50 gel filtration, the duplex DNA was precipitated with ethanol. The hairpin loop in the duplex DNA was cleaved with the aid of the enzyme nuclease S1. For this purpose, the precipitated DNA was taken up in S1 buffer (30 mM Na acetate of pH = 4.5, 250 mM NaCl, 1 mM ZnSO₄ and 5% glycerol) and 10-20 units of S1 enzyme. After 45 minutes at 35°C, the reaction was stopped by addition of EDTA to a final concentration of 20 mM. The double-stranded copy-DNA was subsequently size-fractionated by chromatography over Sepharose C1-4B in a buffer comprising 10 mM Tris-HCl of pH - 8.0, 300 mM NaCl and 1 mM EDTA. Only those fractions with copy-DNA molecules larger than 500 base-pairs in length were precipitated with alcohol.

### C) Tailing and transformation.

The enzyme terminal deoxynucleotidyl transferase catalyses the stepwise addition of deoxynucleoside triphosphates to the 3′ end of polynucleotide chains and is frequently used in the cloning of double-stranded DNA copies of mRNAs. Polycytidyl residues were polymerized onto the 3′ end of the cDNA molecules in a reaction mixture comprising 200 mM K cacodylate pH 7.2, 0.1 mM DTT, 0.1 mM CoCl₂, 0.1 mM dCTP and 12.5 µCi of 3H-dCTP. Ten units of terminal transferase were added and the mixture was followed after 30 minutes at 37°C by a phenol/chloroform extraction, Sephadex G-50 chromatography and alcohol precipitation as described above.

Ten micrograms of the plasmid pUC9 (Vieira, J. and Massing, J., Gene 19 (1982), pages 259-268) were digested with the restriction enzyme PstI and provided with polyguanidyl residues as described above, with the proviso, however, that dGTP (+3H-dGTP) was used instead of dCTP. After 1, 2, 5, 10, 15 and 30 minutes samples were taken and their incorporated radioactivity was determined by means of a trichloroacetic acid precipitation. The remainder of the sample in which 15-20 dG residues are added per terminus was used for the annealing reaction. Approximately the same quantities of copy-DNA with dC tails and cloning vector pUC9 provided with dG tails were mixed in annealing buffer (100 mM NaCl, 10 mM Tris-HCl of pH = 7.7 and 1 mM EDTA), heated at 68°C for 5 minutes and, after 2 hours at 57°C, the mixture was cooled to room temperature. The recombinant DNA molecules were subsequently transformed into E. coli strain JM 83 (Vieira, J. and Messing, J., Gene 19, (1982), pages 259-268). The transformed bacteria were plated on LB agar plates containing 100 µg/ml carbenicillin and 40 µg/ml X-gal and were incubated overnight at 37°C. Only the bacteria which did not show any blue coloration contained plasmid pUC9 provided with a copy-DNA insertion. Approximately 2,000 clones were obtained and further analysed for chymosin sequences.

### D) Isolation of chymosin clones.

Each individual clone containing recombinant DNA was transferred to a new LB agar plate (+ 100 µg/ml carbenicillin) which was incubated overnight at 37°C. The colonies were transferred to a GENE SCREEN PLUS transfer membrane (NEN-Dupont), lysed with alkali followed by neutralization with 1 M Tris-HCl of pH = 7.5, such as was first described by Benton, W.D. and Davis, R.W., Science, 196, (1977) 180-182. Both the chromosomal DNA and the recombinant plasmid DNA bind to the membrane. The dried filters were subsequently prehybridized in a 6 x NET solution (1 x NET: 150 mM NaCl, 15 mM Tris-HCl of pH = 8.3 and 1 mM EDTA), 10 x Denhardt buffer (1 x Denhardt: 0.02% Ficoll, 0.02% BSA and 0.02% polyvinylpyrrolidone) and 0.1% SDS for 4-6 hours at 65°C.

Hybridization took place in the same buffer in which a labelled probe was added. The probe used, 5′ TGGTGCCTGTTCATC 3′, is complementary to the nucleotide sequence 602-616 of the calf preprochymosin as published by Harris, T. et al., Nucl. Acids Research, 10, (1982), pages 2177-2187. A one hundred picomole sample was phosphorylated in the presence of 50 µCi γ-32-P-dATP and 10 units of T4 polynucleotide kinase in a buffer consisting of 70 mM Tris-HCl of pH = 7.6 and 10 mM MgCl₂, for 45 minutes at 37°C. Labelled probe was added to the hybridization mixture and incubated overnight at 40°C. The filters were washed in 6 x SSC (1 x SSC: 150 mM NaCl, 15 mM Na citrate) + 0.1% SDS, dried and subsequently autoradiographed in order to locate those colonies which hybridize with the chymosin-specific probe. From a total of about 2,000 clones, 11 positive transformants containing chymosin sequences were obtained.

### E) Characterization of the chymosin plasmid DNA.

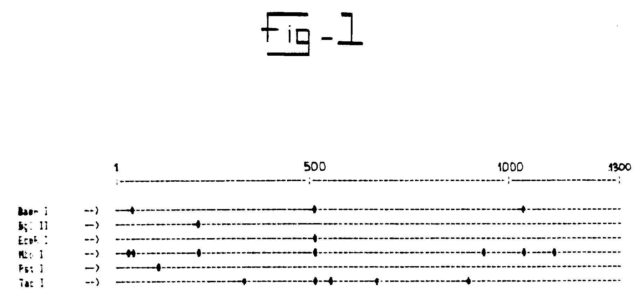

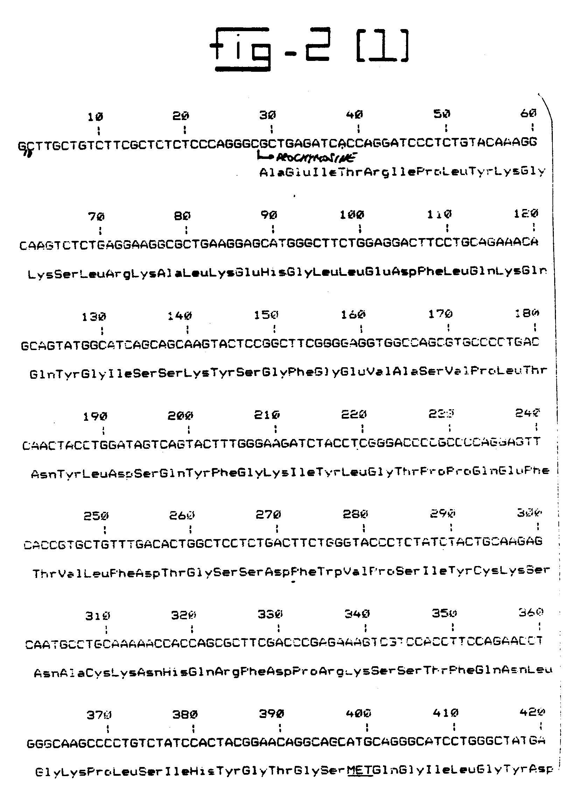

Plasmid DNA was isolated on a small scale from all the 11 positive clones by the method described by Ish Horowitz, D. and Burke, J., Nucleic Acids Research, 9, (1981) pages 2989-2998. The size of the isolated chymosin cDNA was determined by digestion with the restriction enzymes HindIII and SalI, followed by analysis on 1% agarose gels. The insertion size varied between 800 and 1,300 base-pairs. Only those clones with the largest insert (about 1,300 bp), called pNZ400-5 and pNZ400-6, were examined further. Plasmid DNA of the two clones was isolated and cut with various restriction enzymes (see restriction map in Figure 1) in order to determine the position of a number of cleavage sites which are used for subcloning in M13 mp10/mp11 DNA (Messing, J. et al., Nucl. Acids Res. 9, (1981), pages 309-321) for determination of the DNA sequence. The nucleotide sequence was determined with the dideoxy chain termination method developed by Sanger, F. et al., Proc. Natl. Acad. Sci., 74, (1977), pages 5463-5467.

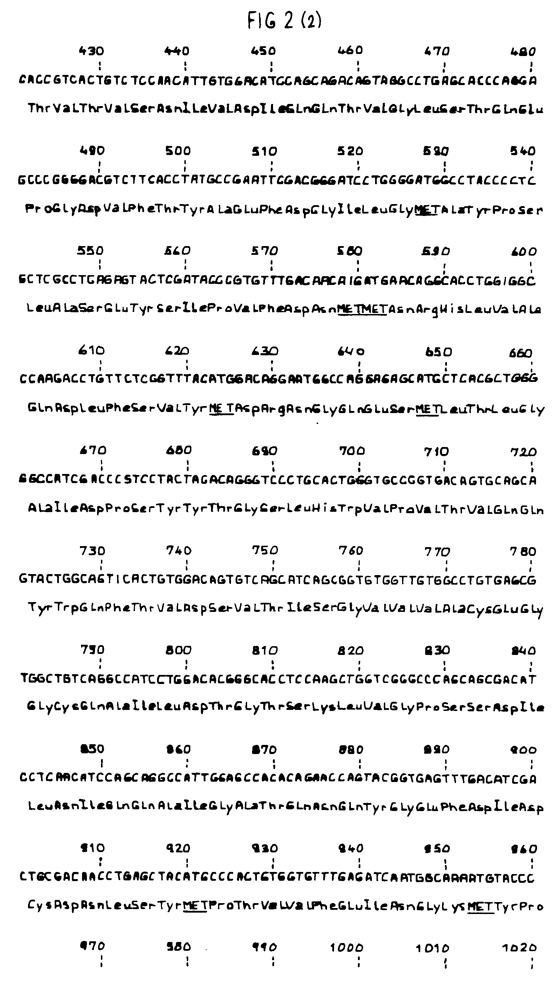

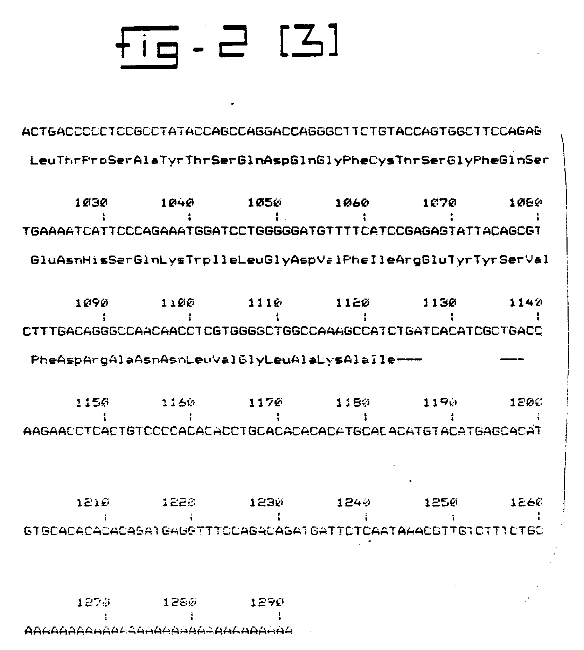

At the same time use was made for the determination of the DNA sequence of the double-stranded DNA sequence method developed by Chen, R. and Seeburg, P., DNA 4, (1985), pages 165-170 and adapted for use of the enzyme reverse transcriptase by Graham, A. et al., FOCUS 8, (1986), pages 4-5. DNA primers used for sequence determination were synthesized by a SAM-ONE synthesizer (New Brunswick Scientific) without further purification. The result of the DNA nucleotide sequence determination is represented in Figure 2. The above amino acid sequence corresponds to the sequence of the chymosin B form published by Foltmann, B. et al., J. of Biol. Chem. 254, (1979), pages 8447-845.

### G) Location of the proteinase gene in the strain S. cremoris SK11.

The S. cremoris SK11 strain SK112 (which is deposited at the Central Bureau for Mould Cultures, The Netherlands, under No. CBS 349.87) contains 3 plasmids named pSK111, pSK113 and pSK118 having molecular weights of 48, 28 and 6 Mdal, respectively. By allowing cells to grow in the presence of acriflavine (concentration of 5 µg/ml) it was possible to allow plasmid pSK111 to disappear from the culture, called curing, which resulted in strain SK1128. This strain does not show, however, any proteolytic activity. The fact that pSK111 codes for proteinase activity was further demonstrated by conjugation experiments. Transfer of pSK111 to a proteinase-deficient strain resulted in the production of proteinase activity (Vos, W.M. de, FEMS Microbiol. Letters 23, (1984), pages 175-179, Vos, W.M. de and Davies, F.L., Third European Congress on Biotechnology, Verlag Chemie, 3, (1984), pages 201-206.

### H) Cloning of the S. cremoris SK112 proteinase gene.

Plasmid DNA was isolated from S. cremoris SK112 according to a method described by Anderson, D.G. and McKay, L.L., Appl. Environ. Microbiol. 46, (1983), page 549. The individual plasmids were separated by electrophoresis of about 50 µg of plasmid DNA on a 1% low-gelling temperature (LGT) agarose gel (Biorad). The 78 kb plasmid pSK111 was cut out and processed as instructed by the manufacturer of the LGT agarose. The pSK111 DNA was digested under partial conditions with Sau3A into fragments of about 10-15 kb which was subsequently cloned in the BamHI site of the bacteriophage vector EMBL3 in E. coli Q359 (Frischauf, H. et al., J. Mol. Biol. 170, (1983), page 827). About 100 recombinant phages which can multiply in this host (spi-) were screened for the production of proteinase with the aid of a so-called gene expression kit (Boehringer). For this, use was made of anti-proteinase antibodies produced by immunization of a rabbit with cell-wall proteinase isolated from S. cremoris SK112 (Hugenholtz, J., Appl. Environ. Microbiol. 48, (1984), page 1105). Insertion DNA present in five recombinant phages, which in E. coli led to the synthesis of proteinase, was isolated and characterized with the aid of suitable restriction enzyme digestions. Various restriction enzyme fragments were isolated from the pSK111 DNA cloned in these phages and were subsequently cloned with the aid of lactobacillus-specific vectors in the proteinase-deficient S. lactis strain MG1363 (Gasson, M.J., J. Bacteriol. 154, (1983), page 1), as described in more detail by Vos, W.M., in Molecular Engineering in the European Community, E. Magnien Ed. (1986), pages 465-472, M. Nijhoff, Dordrecht. The physical maps of the vectors used are shown in Figure 3. All the plasmid vectors are based on the vector pNZ12 containing the pSH71 replicon which is capable of replication in lactic acid bacteria such as S. lactis and L. casei as well as in E. coli, B. subtilis and S. aureus (see de Vos, W.M. and Simons, A.F.M., EP-A 86,202,061.7). The plasmid pNZ121 is constructed by changing the orientation of the 2.7 kb TaqI fragment containing the Cm and Km resistance genes. By removing the Km resistance gene from pNZ121 by means of a SalI and MspI digestion, providing the fragment left behind with blunt ends using Klenow polymerase and circularizing with T4 ligase, the plasmid pNZ122 was constructed.

All plasmid derivatives contain a unique SalI restriction enzyme site and possess the same host range as pNZ12. However, while pNZ12 replicates with a high copy number in lactic acid bacteria pNZ121 and pNZ122 possess a low copy number in these bacteria. It was found on cloning the SK112 proteinase gene that only the low copy number vectors pNZ121 and pNZ122 are capable of maintaining the intact gene with regulator regions in S. lactis MG1363. One of the recombinant plasmids is called pNZ511 and consists of an about 6 kb fragment provided with SalI sites which codes for proteinase production, cloned in the vector pNZ122 (see Figure 4). pNZ511 was capable of completely complementing the proteinase deficiency of S.lactis MG1363 (wild-type growth in milk) and coded for a proteinase activity entirely secreted into the medium and having the same specificity relative to α-, β- and k-casein as the S. cremoris SK112 proteinase. The direction of the proteinase gene and the position of the regulator region of this gene were determined by deletion studies of the 6 kb insert in pNZ511 (for details see de Vos, W.M. in Biomolecular Engineering in the European Community, E. Magnien, Ed. (1986), pages 465-472, M. Nijhoff, Dordrecht).

It was noteworthy that the proteinase encoded by pNZ511 was completely secreted by S. lactis. It will be obvious to a person skilled in the art that this indicates that the proteinase gene contains sequences which are necessary for secretion and that these sequences can be used for the development of so-called secretion vectors.

### I) Determination of the DNA nucelotide sequence of the regulator region and the structural region of the proteinase gene of strain S. cremoris SK112.

Plasmid pNZ511 was digested with the restriction enzyme ClaI, and the small 400 bp fragment was isolated from 1% agarose gel by means of electroelution. This ClaI fragment was subsequently ligated with M13 mp10 DNA cut with the restriction enzyme AccI. Single-strand phage DNA of white plaques was isolated as described by Messing, J. et al., Nucl. Acids Res. 9, (1981), pages 309-321, and the DNA nucleotide sequence was determined by the method of Sanger (Proc. Natl. Acad. Sci. 74, (1977), pages 5463-5467). The result is represented in Figure 5. The fragment is very rich in AT (73%) and shows a number of interesting characteristics. Base sequences which show extensive homologies with the consensus promoter sequence for Bacillus subtilis and Escherichia coli consisting of a -35 region: TTGACA and a -10 region: TATAAT, as described by Hawley, E. and McClure, W., Nucl. Acids. Res. 11, (1983), pages 2237-2255, can be identified in both strands. The presence of 5 successive TATAAT boxes is noteworthy. In addition, a number of direct and inverted repeats having a length of 10 to 15 nucleotides and even a palindromic sequence of 20 nucleotides are detectable in this TATAAT region. This region is furthermore capable of forming a stable hairpin loop structure with a stem of 14 nucleotides and a ΔG value of 15.2 kcal (Figure 6). Downstream of this TATAAT-rich region, 2 ATG initiator codons are detectable. In particular the second ATG codon is preceded by a base sequence which shows extensive complementarity with the 3' end of 16S rRNA of Streptococcus lactis which has recently been determined by Ludwig, W. et al., J. of General Microbiology 131, (1985), pages 453-551. This Shine and Dalgarno domain (Proc. Natl. Acad. Sci. USA 71, (1974), pages 1342-1346) has a ΔG value of -14.4 kcal.

The base sequence upstream and downstream of this ClaI fragment was determined by cloning of the downstream ClaI-SalI fragment (see Figure 4) in suitable overlapping restriction enzyme fragments in M13 mp10/mp11 DNA and the approximately 700 base-pair upstream ClaI-SalI fragment (see Figure 4) in M13 mp10 DNA cut with AccI/SalI and represented in Figure 7a, 7b. It is also evident from this sequence that the postulated promoter sequence located upstream is also followed by an ATG initiator codon and a base sequence which has extensive complementarity with the 3' end of 16S rRNA and has a ΔG value of -11.6 kcal. The derived amino acid sequence of both the downstream and the upstream open reading frame in each case shows great correspondence with a signal peptide which is characteristic for secreted proteins (Watson, M., Nucl. Acids Res. 12, (1984), pages 5145-5164 and Heyne, G. von, J. Mol. Biol. 184, (1985), pages 99-105).

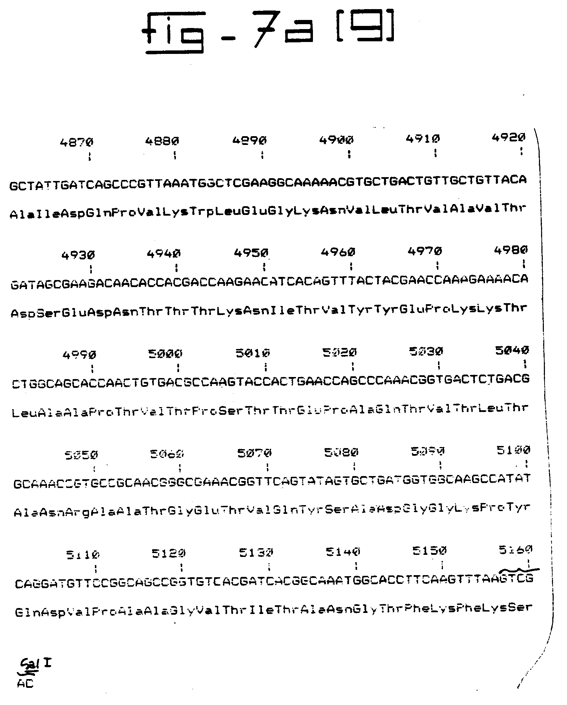

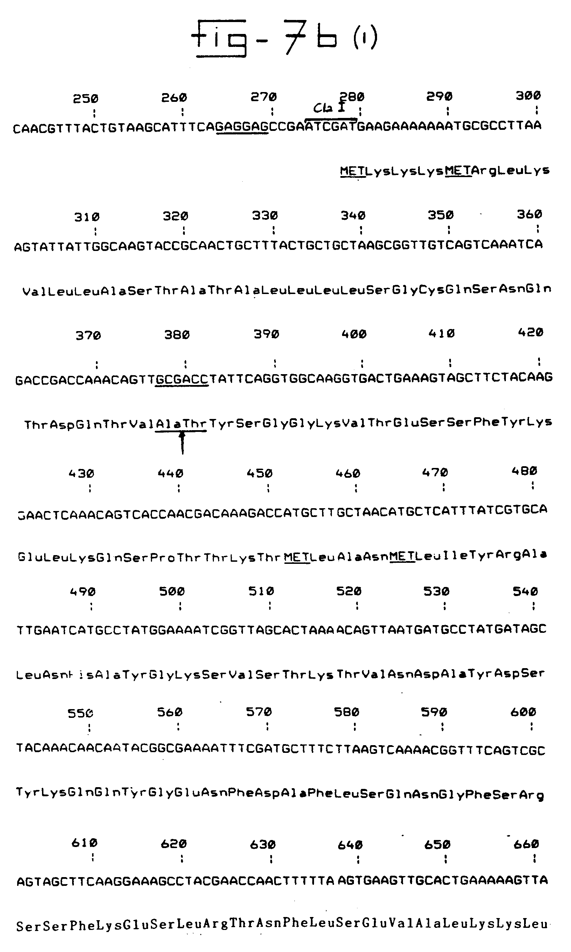

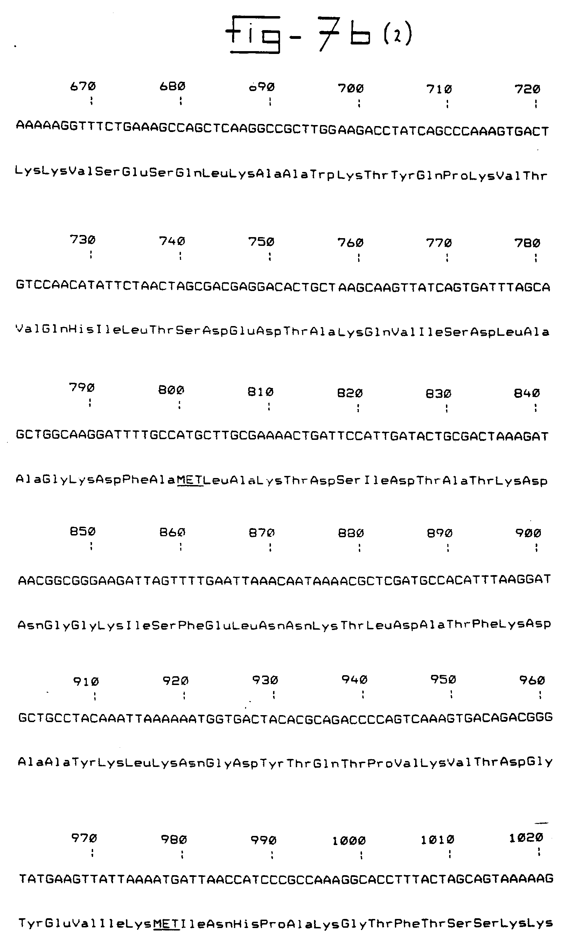

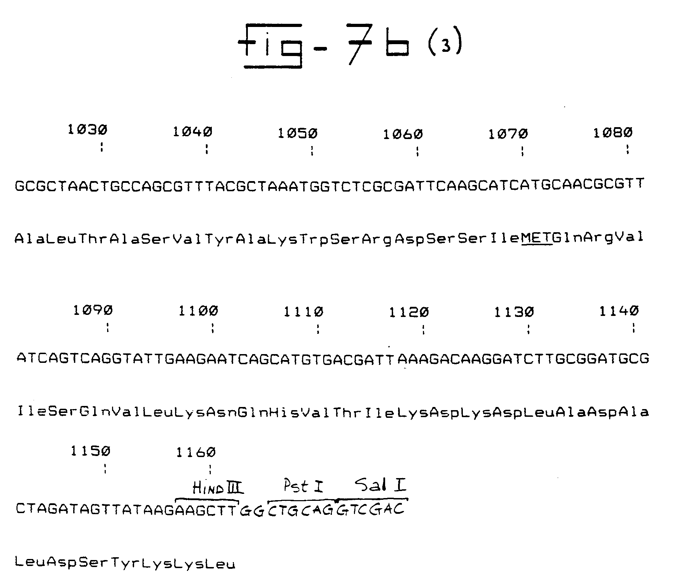

In the case of the open, downstream reading frame a possible signal cleavage site is located between amino acid 33 and 34 (Ala↓Ala) while in the case of the upstream reading frame this is very probably located between residue 34 and 35 (Ala↓Thr). In addition, a gas-phase sequencer (Applied Biosystems) was used to determine the amino-terminal end of the SK112 proteinase, purified from the cell wall, according to a method described by Exterkate, F. and De Veer, G., Appl. Environ. Microbiol. 49, 328-332 (1985). The preparation was analysed on SDS polyacrylamide gel according to Laemmli, Nature 227, 680-685 (1970) and blotted according to a method described by Kerckhove, J. van den et al., Eur. J. Biochem. 152, 9-19 (1986). The amino acid sequence obtained was Asp-Ala-Lys-Ala-Asn-Ser-Meth-Ala-Asn-Val. This amino acid sequence is located at position 188-197 of the protein sequence derived from the DNA sequence. It may be concluded from this that the SK112 proteinase is very probably synthesized as a pre-pro-protein in analogy to serine proteases from B. subtilis (Jacobs, M. et al., Nucl. Acids Res. 13, 8913-8926, (1985)). The functionality of the presumed promoter sequence and ribosome binding site of the downstream open reading frame was demonstrated in Escherichia coli by means of lacZ fusions. Double-stranded replicative DNA of the recombinant M13 phage in which the unique ClaI proteinase fragment of strain S. cremoris SK11 has been inserted was digested with the restriction enzyme HindIII. With the aid of the enzyme DNA polymerase I (Klenow) the protruding ends were filled in, and this was followed by a digestion with the restriction enzyme BamHI. The blunt BamHI fragment thus obtained was subsequently ligated with plasmid pMC1403 (Casadaban, M. et al., J. of Bact. 143, (1980), pages 971-980) which was cut with the restriction enzymes SmaI and BamHI according to the instructions of the supplier. Competent Escherichia coli MC1061 cells were transformed with the ligation mixture, and a large number of blue colonies was obtained. In all the blue transformants, the ClaI protinease fragment appeared to be coupled in phase with the lacZ gene of plasmid pMC1403. A β-galactosidase activity of about 15 µmol/mg protein measured according to a method described by Miller, J., (1972), in Experiments in Molecular Genetics, Cold Spring Harbor Laboratory Press N.Y., was detectable. E. coli cells transformed with the recombinant plasmid (called pNZ290) led to the synthesis of a β-galactosidase fusion protein accounting for approximately 5% of the total cellular protein synthesis (Figure 8).

In order to examine the synthesis of this β-galactosidase fusion protein under the control of the downstream proteinase promoter in Streptococcus lactis, the plasmid pNZ290 was provided with an origin of replication region specific for lactic acid Streptococci. Equal quantities of linearized (incubation with the restriction enzyme SalI) pNZ290 DNA and linearized pNZ121 DNA (Figure 3) were incubated in the presence of the enzyme T4 ligase and transformed into S. lactis MG1363 protoplasts according to a method described by Gasson, M., FEMS Microbiology Letters 9, (1980), pages 99-102 and Wright, A. von et al., Appl. and Environ. Microbiol. 50, (1985), pages 1100-1102.

Transformants were tested for β-galactosidase activity, and positive transformants were capable of synthesizing a β-galactosidase fusion protein having an activity of ± 3 µmol/min/mg protein measured according to a method described earlier.

### J) Fusion of the proteinase promoter to the genetic information of prochymosin.

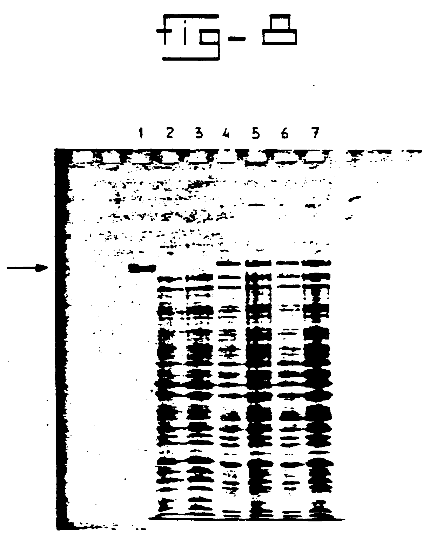

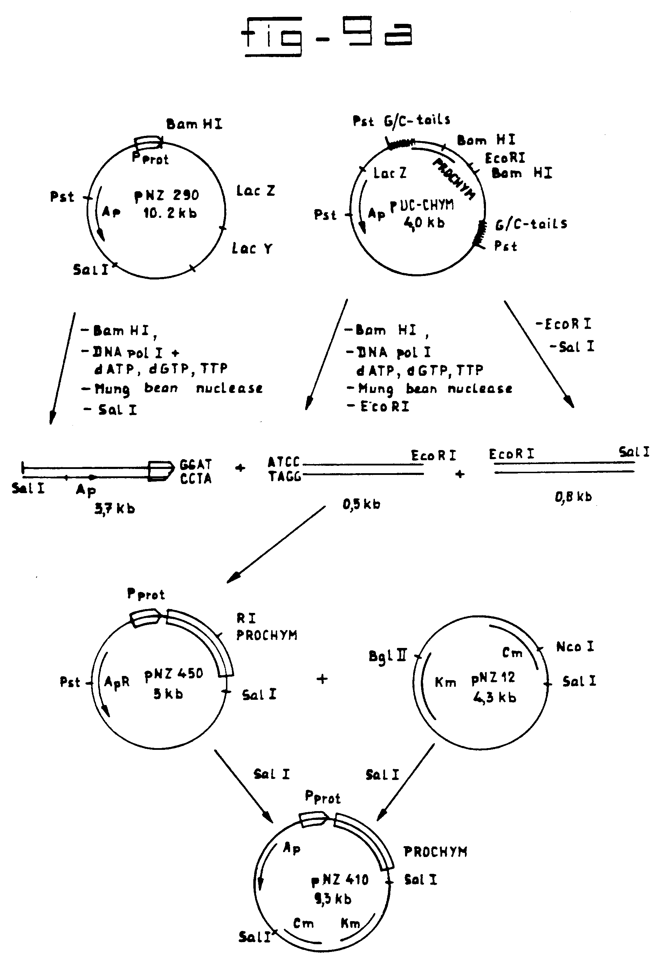

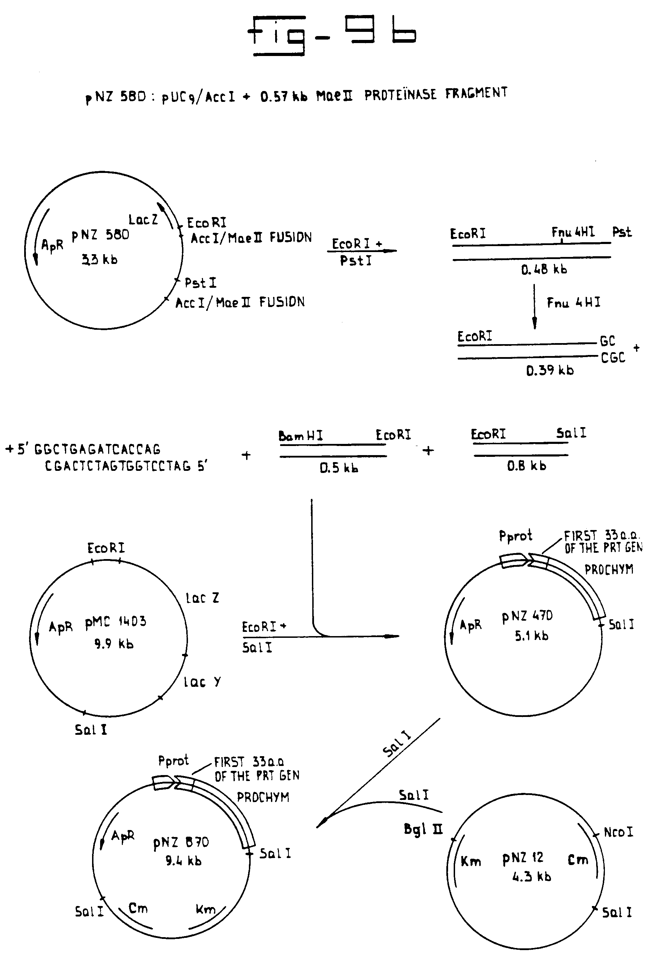

Figure 9a diagrammatically shows the construction of a plasmid for the expression of prochymosin. The prochymosin expression plasmid pNZ450 was constructed by incubating plasmid pNZ290 with the restriction enzyme BamHI, treating this with the enzyme DNA polymerase I (Klenow) in the presence of only 3 deoxynucleosides, specifically dATP, dGTP and TTP, followed by a mung bean nuclease treatment in order to remove the remaining G residue, with the result that a blunt end was produced. All the enzymatic reactions were carried out under conditions as described by the supplier. The linear fragment obtained was subsequently incubated with the restriction enzyme SalI, and the smallest DNA fragment having a size of about 4.0 kb was isolated after fractionation on a 1% agarose gel by means of electroelution. The prochymosin-containing pNZ400-5 DNA was digested with the restriction enzyme BamHI, treated with the enzyme DNA polymerase I (Klenow) in the presence of only 3 deoxynucleosides, specifically dATP, dGTP and TTP, followed by a mung bean nuclease treatment such as described above. This mixture was then incubated with the restriction enzyme EcoRI, and the small 460 base-pair amino-terminal section of prochymosin was isolated by fractionation on a 4% polyacrylamide gel and then by means of electroelution.

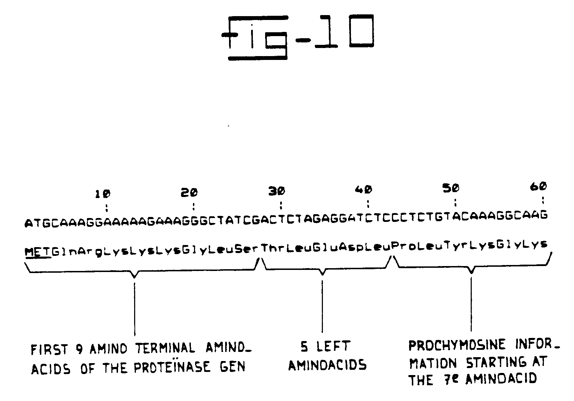

Both the 4.0 kb proteinase promoter fragment and the two chymosin fragments (0.5 and 0.8 kb, respectively) were ligated to each other with the aid of the enzyme T4 DNA ligase. After transformation of the ligation mixture into E. coli strain MC1061, the sequence of a number of selected transformants was determined in order to confirm the correct transformation of the various fragments. A coupling in phase between the reading frame of the protinease gene and the Δ7 prochymosin information appears to have been brought about in the plasmid pNZ450-27 (see Figure 10). Plasmid pNZ450-27 was incubated with the restriction enzyme SalI and ligated with linear pNZ12 DNA (see Figure 3) obtained after incubation with the enzyme SalI. The ligation mixture was transformed into S. lactis strain MG1363 according to a method described by Gasson, M., FEMS Microbiology 9, (1980), pages 99-102 and Wright, A. von et al., Appl. and Environ. Microbiol. 50, (1985), pages 1100-1102. Selected chimeric DNA-containing transformants, called pNZ410 (Figure 9a) were tested for prochymosin synthesis by means of Western blotting.

Figure 9b is a schematic illustration of the construction of a plasmid for the secretion of prochymosin. The prochymosin secretion plasmid pNZ470 was constructed by at first subcloning the 0.57 kb MaeII fragment containing the complete Sk11 proteinase promotor sequence (position 54, Fig. 5) and the first 94 amino-terminal amino acids of the proteinase gen (position 761, Fig. 7a) from pNZ511 in the AccI site of pUC9 resulting in pNZ580 (see Fig. 9b). The 0.48 kb DNA fragment, obtained after an EcoRI/PstI double digestion of pNZ580, was isolated by means of electro-elution after fractionating on a 1% agar gel. This fragment contains the complete regulator sequence of the Sk11 proteinase gen until amino acid 62. Then this fragment was incubated with the enzym Fnu 4HI. This fragment had more Fnu 4HI-sites but the first Fnu 41 HI-site from the 5′-end is lain between the amino acids 33 and 34 (position 578 in Fig. 7a). Subsequently the synthetic double strand DNA-fragment 
5' G GCT GAG ATC ACC AG
CGA CTC TAG TGG TC CTAG
and the two chymosin DNA-fragments (size: 0.5 kb and 0.8 kb; see Fig. 9a) were ligated to the 0.39 kb DNA-fragment by means of enzym T4 DNA ligase. The synthetic DNA-fragment codes for the first six aminoterminal amino acids of the prochymosin. The resulting 1.7 kb fragment was then ligated to the vector pMC1403 (Casadaban Metal J. of Bact. 143, 1980, 971-980), which was cut with the restriction enzymes EcoRI and SalI and transformed to the E. coli strain MC1061. In the obtained plasmid pNZ470 the prochymosin information was coupled in phase to amino acid 33 of the proteinase gen.

Plasmid pNZ470 was further incubated with the restriction enzym SalI and ligated with linear pN12 obtained after incubation with SalI. The ligation mixture pNZ870 was transformed to the S. lactis strain MG1363 according to a previously described method, and the transformants were tested for prochymosin secretion by means of Western blotting.

### L) Western blotting.

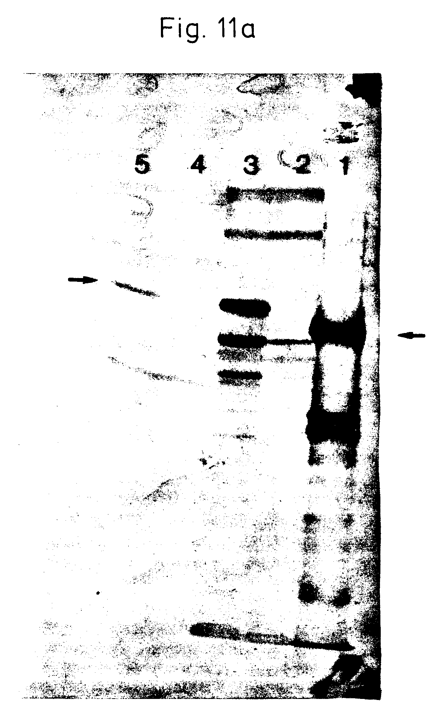

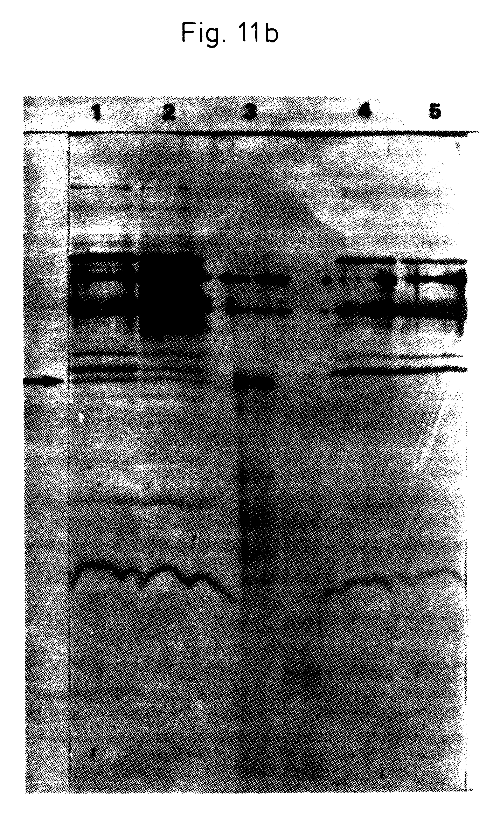

A logarithmically growing culture of strain S. lactis MG1363 containing pNZ410 in M17 medium + glucose (0.5%) + chloramphenicol (10 µg/ml) was spun down and taken up in a buffer consisting of 15% sucrose, 50 mM Tris-HCl of pH = 8.0 and 10 mg/ml lysozyme, and incubated for 30 minutes at 37°C. An identical volume of 2 x SDS sample butter (Laemmli, U., Nature 227, (1970), pages 680-685) was added and heated for 10 minutes in a boiling water bath. After analysis on 12.5% polyacrylamide gel according to Laemmli, followed by blotting on a nitro-cellulose filter, the filter was incubated with anti-bodies against chymosin. The filter was subsequently incubated with peroxidase-labelled goat anti-rabbit anti-bodies and dyed with 4-chloro-1-naphthol according to a method described by the supplier (BRL). A pronounced coloration of a protein band having a molecular weight of 40,000 D was noticeable in a S. lactis MG1363 lysate containing plasmid pNZ410 by comparison with a lysate containing plasmid pNZ12. This band has a somewhat slower migration rate in the SDS gel (analytical grade) than mature chymosin (Figure 11).

A logarithmically growing culture of strain S. lactis MG1363 containing pNZ870 in a medium consisting of 5% UF-whey permeate + casiton (1%) + β-glycerophosphate (1.9%) + chloramphenicol (10 µg/ml) was spun down and the supernatant was dialysed for 16 hours against double distilled water. After lyophylisation of the dialysate and taking up the dry material in buffer a quantity, corresponding to 1 ml supernatant was analysed on 12.5% polyacrylamide gel according to Laemmli. After blotting on a nitrocellulose filter, the filter was incubated with antibodies against chymosine. The filter was subsequently incubated with peroxidase-labelled goat anti-rabbit antibodies and dyed with 4-chloro-1-naphthol. A pronounced coloration of a protein band having the exact size of the prochymosin was noticeable in the supernatant of a S. lactis MG1363 culture containing plasmid pNZ870.

### LEGENDS

- Figure 1:: Restriction enzyme digestion map of the cloned prochymosin insert.
- Figure 2:: DNA sequence of the cloned chymosin and the amino acid sequence derived therefrom.
- Figure 3:: Physical map of the plasmids pSH71, pNZ121, pNZ122 and pNZ12.
- Figure 4:: Physical map of the plasmid pNZ511; this plasmid was produced after cloning of the 6 kb proteinase fragment in the vector pNZ122.
- Figure 5:: DNA sequence of the ClaI fragment of the proteinease gene of Streptococcus cremoris SK112, cloned in the AccI site of M13 mp10.
- Figure 6:: Hairpin loop structure in the promoter region.
- Figure 7a:: DNA sequence of the downstream ClaI-SalI fragment of the proteinase gene of pSKIII in S. cremoris SK112.
- Figure 7b:: DNA sequence of the upstream ClaI-SalI fragment of the proteinase gene of pSK111 of S. cremoris SK112.
- Figure 8:: Polyacrylamide gel of cell lysates containing plasmid pMC1403 (2 and 3) and pNZ290 (4-7). Lane 1:5 µg β-galactosidase.
- Figure 9a:: Diagrammatic representation of the construction of plasmids which lead to the synthesis of prochymosin (pNZ450 and pNZ410).
- Figure 9b:: Diagrammatic representation of the construction of plasmids which lead to the secretion of prochymosin (pNZ870).
- Figure 10:: DNA sequence of the in phase fusion between the amino-terminal end of the proteinase gene and the prochymosin information.
- Figure 11a:: Western blot of cell lysates of E. coli and Streptococcus lactis
1) 5 µg mature chymosin
2) E. coli MC1061 cell extract + pNZ12
3) E. coli MC1061 cell extract + pNZ450
4) S. lactis MG1363 cell extract + pNZ12
5) S. lactis MG1363 cell extract + pNZ410

## Claims

1. Isolated DNA fragment containing a lactic acid bacterium-specific regulator region or the proteinase gene as shown in Figure 5 containing the ClaI fragment located on the plasmid pSK111 of the strain S. cremoris SK112, deposited at the Central Bureau for Mould Culture, The Netherlands under No. CBS 349.87 or a mutated form thereof having essentially the same activity.

2. Isolated DNA fragment according to Claim 1, supplemented with the base sequence or functional parts thereof located downstream of the ClaI site as shown in Figure 7a.

3. Isolated DNA fragment according to Claim 2, comprising the ClaI fragment as shown in Figure 5, supplemented downstream with the DNA fragment illustrated in Figure 7a(1) and located between the ClaI site present in the second line ad the Ala-Ala-sequence, present in the third line of Figure 7a(1).

4. Isolated DNA fragment according to Claim 1, supplemented with the base sequence located upstream of the ClaI site or functional parts thereof as shown in Figure 7b.

5. Recombinant plasmid containing a DNA fragment according to one or more of Claims 1-4.

6. Recombinant plasmid according to Claim 5 containing a replicon derived from lactic acid bacteria.

7. Recombinant plasmid according to Claim 5 containing a replicon derived from S.lactis plasmid pSH71.

8. Recombinant plasmid according to Claim 7 containing a plasmid vector pNZ121 or pNZ122 as shown in Figure 3, wherein pNZ121 is obtainable by changing the orientation of the 2.7 Kb TaqI fragment containing the Cm and Km resistance genes and pNZ122 is obtainable by removing the Km resistance gene from pNZ121 by means of a SalI and MspI digestion, providing the fragment left behind with blunt ends using Klenow polymerase and circularizing with T4 ligase.

9. Recombinant plasmid according to one or more of Claims 5-8 in which a gene coding for a protein is connected to at least the regulator region according to one or more of Claims 1-4.

10. Recombinant plasmid according to Claim 9, containing a gene coding for prochymosin, preprochymosin, pseudochymosin and chymosin, respectively.

11. Recombinant plasmid according to Claim 9, containing a gene coding for β-galactosidase.

12. Recombinant plasmid according to Claim 9, containing a gene coding for proteinase as shown in Figure 4.

13. Lactic acid bacterium containing a plasmid according to one or more of Claims 5-12.

14. Lactic acid bacterium which is a Lactobacillum such as L.casei, containing a plasmid according to one or more of Claims 5-12.

15. Lactic acid bacterium which is a lactic acid Streptococcus such as Streptococcus lactis and Streptococcus cremoris containing a plasmid according to one or more of Claims 5-12.

16. Lactic acid bacterium of the strain Streptococcus lactis MG1363 containing a plasmid according to one or more of Claims 5-12.

## Patentansprüche

1. Isoliertes DNA-Fragment, das eine Milchsäurebakteriumspezifische Regulatorregion des Proteinasegens enthält, wie in Figur 5 gezeigt, die das Cla^{I}-Fragment enthält, angeordnet auf dem Plasmid pSK111 des Stammes S. cremoris SK112, der hinterlegt ist bei dem Central Bureau for Mould Culture, The Netherlands, unter No. CBS 349,87, oder auf einer mutierten Form davon, die im wesentlichen dieselbe Aktivität aufweist.

2. Isoliertes DNA-Fragment nach Anspruch 1, ergänzt um die Basensequenz oder mit funktionellen Teilen davon, die stromabwärts von der ClaI-Stelle angeordnet sind, wie in Figur 7a gezeigt.

3. Isoliertes DNA-Fragment nach Anspruch 2, umfassend das ClaI-Fragment, wie in Figur 5 gezeigt, das stromabwärts ergänzt ist um das in Figur 7a(1) gezeigten DNA-Fragment und sich zwischen der ClaI-Stelle, die in der zweiten Zeile vorhanden ist und der Ala-ala-Sequenz befindet, die in der dritten Zeile von Figur 7a(1) vorhanden ist.

4. Isoliertes DNA-Fragment nach Anspruch 1, ergänzt um die Basensequenz, die stromaufwärts von der ClaI-Stelle angeordnet ist, oder mit funktionellen Teilen davon, wie in Figur 7b gezeigt.

5. Rekombinantes Plasmid, das ein DNA-Fragment nach einem oder mehreren der Ansprüche 1-4 enthält.

6. Rekombinantes Plasmid nach Anspruch 5, das ein sich von Milchsäurebakterien ableitendes Replicon enthält.

7. Rekombinantes Plasmid nach Anspruch 5, das ein sich von dem S. lactis-Plasmid pSH71 abgeleitetes Replicon enthält.

8. Rekombinantes Plasmid nach Anspruch 7, das einen Plasmidvektor pNZ121 oder pNZ122 enthält, wie in Figur 3 gezeigt, worin pNZ121 zugänglich ist, indem die Orientierung des 2,7 kb TaqI Fragment, das die Cm- und Km-Resistenzgene enthält, verändert wird, und worin pNZ122 zugänglich ist, indem das Km-Resistenzgen von pNZ121 mittels eines SalI- und MspI-Verdaus entfernt wird, wobei das zurückgelassene Fragment unter Verwendung von Klenow-Polymerase mit glatten Enden versehen wird und indem mit T4-Ligase ein Ringschluß durchgeführt wird.

9. Rekombinantes Plasmid nach einem oder mehreren der Ansprüche 5-8, worin ein Gen, das für ein Protein kodiert, wenigstens mit der Regulatorregion nach einem oder mehreren der Ansprüche 1-4 verknüpft ist.

10. Rekombinantes Plasmid nach Anspruch 9, das ein Gen enthält, das für Prochymosin, Preprochymosin, Pseudochymosin bzw. Chymosin kodiert.

11. Rekombinantes Plasmid nach Anspruch 9, das ein Gen enthält, das für β-Galactosidase kodiert.

12. Rekombinantes Plasmid nach Anspruch 9, das ein Gen enthält, das für Proteinase kodiert, wie in Figur 4 gezeigt.

13. Milchsäurebakterium, das ein Plasmid nach einem oder mehreren der Ansprüche 5-12 enthält.

14. Milchsäurebakterium, das Lactobacillus heißt, wie L. casei, das ein Plasmid nach einem oder mehreren der Ansprüche 5-12 enthält.

15. Milchsäurebakterium, das zu den Milchsäurestreptokokken gehört, wie Streptococcus lactis und Streptococccus cremoris, das ein Plasmid nach einem oder mehreren der Ansprüche 5-12 enthält.

16. Milchsäurebakterium des Stamms Streptococcus lactis MG1363, das ein Plasmid nach einem oder mehreren der Ansprüche 5-12 enthält.

## Revendications

1. Fragment d'ADN isolé contenant une région régulatrice, spécifique d'une bactérie lactique, du gène de protéinase comme représenté sur la figure 5, contenant le fragment ClaI localisé sur le plasmide pSK111 de la souche S. cremoris SK112 déposée au Central Bureau for Mould Culture, Pays-Bas, sous le n° CBS 349.87, ou forme mutée de celui-ci ayant essentiellement la même activité.

2. Fragment d'ADN isolé, selon la revendication 1, complété par une séquence de bases, ou des parties fonctionnelles de celle-ci, localisée en aval du site ClaI comme représenté sur la figure 7a.

3. Fragment d'ADN isolé, selon la revendication 2, comprenant le fragment ClaI représenté sur la figure 5, complété en aval par le fragment d'ADN représenté sur la figure 7a(1) et localisé entre le site ClaI présent dans la seconde ligne et la séquence Ala-Ala présente dans la troisième ligne de la figure 7a(1).

4. Fragment d'ADN isolé, selon la revendication 1, complété par la séquence de bases localisée en amont du site ClaI ou des parties fonctionnelles de celle-ci comme représenté sur la figure 7b.

5. Plasmide recombinant contenant un fragment d'ADN selon une ou plusieurs des revendications 1 à 4.

6. Plasmide recombinant selon la revendication 5, contenant un réplicon provenant de bactéries lactiques.

7. Plasmide recombinant selon la revendication 5, contenant un réplicon provenant du plasmide pSH71 de S. lactis.

8. Plasmide recombinant selon la revendication 7, contenant un vecteur plasmidique pNZ121 ou pNZ122 tel que représentés sur la figure 3, pNZ121 pouvant être obtenu par changement de l'orientation du fragment TaqI de 2,7 kb contenant les gènes de résistance Cm et Km, et pNZ122 pouvant être obtenu par élimination du gène de résistance Km de pNZ121 au moyen d'une digestion par SalI et MspI, apport d'extrémités franches au fragment restant, à l'aide du fragment de Klenow de la polymérase, et circularisation à l'aide de ligase de T₄.

9. Plasmide recombinant selon une ou plusieurs des revendications 5 à 8, dans lequel un gène codant pour une protéine est connecté à au moins la région régulatrice selon une ou plusieurs des revendications 1 à 4.

10. Plasmide recombinant selon la revendication 9, contenant un gène codant pour la prochymosine, la préprochymosine, la pseudochymosine et la chymosine, respectivement.

11. Plasmide recombinant selon la revendication 9, contenant un gène codant pour la β-galactosidase.

12. Plasmide recombinant selon la revendication 9, contenant un gène codant pour une protéinase comme représenté sur la figure 4.

13. Bactérie lactique contenant un plasmide selon une ou plusieurs des revendications 5 à 12.

14. Bactérie lactique qui est un Lactobacillus tel que L. casei, contenant un plasmide selon une ou plusieurs des revendications 5 à 12.

15. Bactérie lactique qui est un streptocoque lactique tel que Streptococcus lactis et Streptococcus cremoris, contenant un plasmide selon une ou plusieurs des revendications 5 à 12.

16. Bactérie lactique de la souche Streptococcus lactis MG1363, contenant un plasmide selon une plusieurs des revendications 5 à 12.
